# EUROPEAN PATENT APPLICATION

(11) **EP 2 287 604 A1**
(43) Date of publication of application: **23.02.2011**
(21) Application number: 09762521.4
(22) Date of filing: 11.06.2009
(51) Int. Cl.: G01N 30/88, G01N 27/447, G01N 27/62, G01N 33/53

(54) **METHOD FOR EXAMINATION OF ALZHEIMER S DISEASE**

(30) Priority: 13.06.2008 JP 2008155433
(71) Applicant: Eisai R&D Management Co., Ltd., Tokyo 112-8088 (JP)
(72) Inventor: MYINT, Khin Than, Tsukuba-shi Ibaraki 300-2635 (JP); ODA, Yoshiya, Tsukuba-shi Ibaraki 300-2635 (JP); ASAI, Naoki, Tsukuba-shi Ibaraki 300-2635 (JP)
(74) Representative: Dean, John Paul
(86) International application number: PCT/JP2009/060655
(87) International publication number: WO 2009/151091

(57) **Abstract**

A method for examining Alzheimer's disease comprising (1) the measurement step of measuring an amount of lidocaine or a metabolite thereof in a cerebrospinal fluid derived from a lidocaine-administered test subject, and (2) the determination step of determining whether the test subject has Alzheimer's disease or not on the basis of the measured amount.

## Description

### Technical Field

The present invention relates to a method for examining Alzheimer's disease.

### Background Art

Alzheimer's disease (henceforth referred to as AD) is a neurodegenerative disease accompanied by appearance of senile plaques and neurofibrillary change in the brain, and it is pathologically characterized by tangle of neurofibrils in the neurons, pathological accumulation of congophilic fibrous materials produced as neuritic plaques (or senile plaques), and deposition of beta amyloid proteins. What constitutes the core of senile plaques consists of these peptides of about 39 to 42 residues called beta amyloids, and they are excised from amyloid precursor proteins.

Diagnosis of AD is performed mainly on the basis of clinical symptoms and medical interview, and it is confirmed on the basis of cerebral atrophy observed by CT scan or electroencephalogram. Although the tau proteins and phosphorylated tau proteins in the cerebrospinal fluid, soluble beta amyloids, and so forth are also used as the marker, accuracy (sensitivity and specificity) of the diagnosis is still insufficient, and they have not come to be widely used (Non-patent documents 1 to 5).

### Prior Art References

### Non-patent documents

Non-patent document 1: Clark CM, Davatzikos C, Borthakur A, Newberg A, Leight S, Lee VM, Trojanowski JQ, Biomarkers for early detection of Alzheimer pathology, Neurosignals, 2008; 16(1):11-8
Non-patent document 2: Reijn TS, Rikkert MO, van Geel WJ, de Jong D, Verbeek MM, Diagnostic accuracy of ELISA and xMAP technology for analysis of amyloid beta (42) and tau proteins, Clin Chem, 2007; 53(5):859-65
Non-patent document 3: Ramani A, Jensen JH, Helpern JA, Quantitative MR imaging in Alzheimer disease, Radiology, 2006; 241 (1) :26-44
Non-patent document 4: Azzazy HM, Mansour MM, Kazmierczak SC, Nanodiagnostics: a new frontier for clinical laboratory medicine, Clin Chem, 2006;52(7):1238-46
Non-patent document 5: Sunderland T, Gur RE, Arnold SE, The use of biomarkers in the elderly: current and future challenges, Biol Psychiatry, 2005 Aug 15;58(4):272-6

### Summary of the Invention

### Object to be Achieved by the Invention

An object of the present invention is to provide a method for examining AD based on use of cerebrospinal fluid, which shows superior sensitivity and specificity.

### Means for Achieving the Object

The inventors of the present invention investigated substances found in cerebrospinal fluid of AD patients, then found that lidocaine was detected at an AD patient-specific low concentration, and thus accomplished the present invention.

That is, the specific means for achieving the aforementioned object are as follows.
[1] A method for examining Alzheimer's disease comprising:
   (1) the measurement step of measuring an amount of lidocaine or a metabolite thereof in a cerebrospinal fluid derived from a lidocaine-administered test subject, and
   (2) the determination step of determining whether the test subject has Alzheimer's disease or not on the basis of the measured amount.
[2] The method according to [1], wherein the measurement step is performed by gas chromatography, liquid chromatography, immunoassay, or capillary electrophoresis.
[3] The method according to [1], wherein the measurement step is performed by mass spectrometry.
[4] The method according to [1], wherein the measurement step is performed by liquid chromatography and mass spectrometry.
[5] An examination kit for Alzheimer's diseases for use in the examination method according to any one of [1] to [4], which comprises a reagent and/or an apparatus for quantifying lidocaine.

### Effect of the Invention

A method for examining AD showing superior sensitivity and specificity is provided.

### Modes for Carrying out the Invention

The examination method of the present invention comprises the measurement step of measuring an amount of lidocaine or a metabolite thereof in a cerebrospinal fluid derived from a lidocaine-administered test subject, preferably human, and the determination step of determining whether the test subject has AD or not on the basis of the measured amount.

The cerebrospinal fluid derived from the test subject may be one extracted by a known method such as lumbar puncture. Administration of lidocaine may be similar to that performed for epidural anesthesia in the case of collection of cerebrospinal fluid. When lidocaine anesthesia is employed as the epidural anesthesia for collection of cerebrospinal fluid, the administration of lidocaine is sufficiently attained by the administration of lidocaine for the anesthesia. In general, 200 mg of lidocaine hydrochloride is epidurally administered. The cerebrospinal fluid is extracted while the anesthesia is maintained, and it is generally collected immediately after the lidocaine administration or within 10 minutes after the lidocaine administration.

Lidocaine in the cerebrospinal fluid is a substance known as a local anesthetic. The metabolite of lidocaine in the cerebrospinal fluid is not particularly limited, so long as it is a metabolite of lidocaine existing in the cerebrospinal fluid, and it may be, for example, monoethylglycinexylidide (MEGX).

The method for measuring lidocaine or a metabolite thereof in the cerebrospinal fluid is not particularly limited, and known methods can be used. Moreover, an apparatus for measurement or analysis suitable for implementation of the measurement can be used. Specific examples include chromatography such as gas chromatography (Tsai PS et al., Anesth Analg, 1998 Sep;87(3):601-4, Hattori H et al., J Chromatogr, 1991 Mar 8;564(1):278-82) and liquid chromatography (Chen L et Al., J Pharm Biomed Anal, 2007 Apr 11;43(5):1757-62, Epub 2006 Dec 30, Marttila E et al., Eur J Clin Pharmacol, 1983;25(5):639-41), immunoassay such as radioimmunoassay (Calvo B et al., J Clin Pharmacol, 1995 Apr;35(4):426-31), enzyme immunoassay and fluoroimmunoassay, and capillary electrophoresis (Anderson MS et al., Rapid Commun Mass Spectrom, 2004;18 (22):2612-8, Vera-Candioti L et al., Anal Chim Acta, 2007 Jul 9;595(1-2):310-8). The measurement can also be carried out by mass spectrometry (Kadioglu Y, Atila A., Biomed Chromatogr, 2007 21 (10):1077-82, Maes A, Weiland L, Sandersen C, Gasthuys F, De Backer P, Croubels S, J Chromatogr B Analyt Technol Biomed Life Sci, 2007;852 (1-2):180-7).

As a preferred method, combination of liquid chromatography (LC) and mass spectrometry (MS) (LC/MS) can be mentioned (Chik Z, Lee TD, Holt DW, Johnston A, Tucker AT, J Chromatogr Sci, 2006;44(5): 262-5, Kawano S, Murakita H, Yamamoto E, Asakawa N., J Chromatogr B Analyt Technol Biomed Life Sci, 2003;792(1):49-54).

The cerebrospinal fluid may be subjected to a pretreatment such as a treatment for removal of proteins and desalting depending on the measurement method.

By using amount of lidocaine or a metabolite thereof in the cerebrospinal fluid as a marker, whether the subject has AD or not is determined. For example, when the value of lidocaine or a metabolite thereof is lower than the value of a healthy subject (normal value), the subject can be determined to have AD. Alternatively, presence or absence of AD may be determined by comparison of the amount with a predetermined lower limit of normal value (cutoff value, for example, average of concentrations of healthy subjects of control group - standard deviation).

The present invention also relates to a kit for examination of AD for use in the examination method of the present invention, which comprises a reagent and/or an apparatus for quantifying lidocaine or a metabolite thereof in cerebrospinal fluid.

The reagent and/or apparatus for quantifying lidocaine or a metabolite thereof in cerebrospinal fluid is suitably chosen according to the measurement principle of the measurement step.

For example, when the measurement is performed by chromatography, the kit includes a reagent and/or an apparatus required for a pretreatment of cerebrospinal fluid. Examples of such a reagent and/or apparatus include ultrafiltration filter for removing protein components, desalting column, buffer, internal standard substance, and so forth. When measurement is performed by immunoassay, the kit contains antibodies directed to lidocaine or a metabolite thereof. In this case, besides such antibodies, second antibodies, lidocaine standard solution, diluent, washing solution, substrate for detection, and so forth may be contained.

### Examples

Hereafter, the present invention will be more specifically explained with reference to examples. However, the present invention is not limited to these examples.

### 1. Pretreatment of samples

Cerebrospinal fluids of 17 Alzheimer's disease patients (10 male and 7 female Caucasions) and 17 healthy subjects of similar age, sex and race were used as samples. The samples were collected under epidural anesthesia with lidocaine (lidocaine hydrochloride (standard dose: 200 mg) was hypodermically administered at the puncture site). Protein components were removed from 150 microliters of each cerebrospinal fluid by using an ultrafiltration filter of 5000 NMWL (Ultrafree-MC BIOMAX-5, UFC3BCC00, produced by Millipore). To the cerebrospinal fluid, 25 microliters of a solution containing L-Valine-¹³C₅,¹⁵N or L-Lysine-¹³C₅,¹⁵N as an internal standard substance (0.1% formic acid, 50% methanol solvent) and 800 microliters of a 0.15% hydrochloric acid solution were added. This sample solution was applied to a solid phase extraction column, Oasis MCX (10 mg), produced by Waters. Washing was performed with 1 mL of water first, then with 1 mL of 50% methanol solvent containing 0.1% formic acid, subsequently with 1 mL of water, and with 1 mL of methanol. Then, elution was performed with 1 mL of a mixture of 25% aqueous ammonia and methanol (2:3, v/v), the eluate was dried, the residue was dissolved again in 25 microliters of 50% methanol, and insoluble substances were removed by using a PVDF membrane filter having a pore size of 0.22 micrometer to prepare a sample for LC/MS.

### 2. LC/MS Conditions

As for the material packed in the LC column, Primesep A resin (particle size: 5 micrometers, pore size: 100 angstroms) of SIELC Technologies (Prospect Heights, IL) was packed in a fused silica capillary having an internal diameter of 150 micrometers (internal surface was treated with TC-WAX, produced by GL Sciences, catalog number: 1010-27012) of which tip was tapered to a diameter of about 8 micrometers beforehand with a laser-based puller P-2000 (produced by Sutter) at a height of about 15 cm under a nitrogen gas pressure of about 7 MPa, and this was used as a column. 50% Methanol containing 0.1% formic acid was used as a mobile phase A, and 90% methanol containing 2% trifluoroacetic acid in addition to 0.1% formic acid was used as a mobile phase B. The concentration gradient of the mobile phase B was, from the start of elution, as follows: 0 minute (0%) - 5 minutes (0%) - 25 minutes (25%) -25.1 minutes (50%) - 50 minutes (100%) - 60 minutes (100%), and the flow rate was 1 microliter per minute. Since valine as the internal standard substance was eluted in a period of 5 to 25 minutes, and lysine as the internal standard substance was eluted in a period of 25 to 50 minutes, either one of the internal standard substances was appropriately used for quantification of each substance depending on the elution time. Qstar Pulsar i produced by Applied Biosystems was used in the cation detection mode as the mass spectrometer with an applied voltage of 2400 volts at a DP value of 10 and an IP value of 100 for improving the sensitivity to perform the measurement for the scanning range of 50 to 700 amu.

### 3. Data Analysis

Several hundreds to one thousand and several hundreds of peaks were sorted out by visual inspection for each sample, and it was found that two peaks of m/z 235.17 eluted at about 30 minutes and m/z 207.15 eluted at about 25 minutes were observed for many of healthy subjects, whereas these peaks were observed for a very few of the Alzheimer's disease patients. Further, by the MS/MS measurement, m/z 86.08 and m/z 58.06 were obtained from the former and the latter, respectively, as the product ions. The former was estimated to be that of lidocaine, and the latter was estimated to be that of MEGX, which is a metabolite of lidocaine. Therefore, specimens of these substances were obtained, and measured under the same LC/MS conditions. As a result, the same retention times and mass spectra were obtained, and thus it was confirmed that these peaks were those of lidocaine and MEXG.

The measured values for lidocaine and MEXG in the AD patients and healthy subjects are shown in Table 1. As clearly seen from the results shown in Table 1, in the cerebrospinal fluids, lidocaine and the metabolite thereof, MEXG, were detected at AD patient-specific low concentrations. Further, it was confirmed that, if the peak intensity ratio of lidocaine to lysine not higher than 0.6 is determined to indicate Alzheimer's disease and the same not lower than 0.9 is determined to indicate normality, for example, since 15 patients could be detected among the Alzheimer's disease patients, the sensitivity could be calculated to be 88.2%, and since 14 subjects could be determined to be normal among the normal subjects, the specificity could be calculated to be 82.4%. Thus, it became clear that AD could be diagnosed by measuring lidocaine and MEGX in cerebrospinal fluid.

**[Table 1]**

| AD patient | | | Healthy control | | |
|---|---|---|---|---|---|
| Sample No. | Peak ratio to lysine | | Sample No. | Peak ratio to lysine | |
| | **MEGX** | **Lidocaine** | | **MEGX** | **Lidocaine** |
| **MA2** | 0 | 0.021 | **MC1** | 0.103 | 1.31 |
| **MA3** | 0 | 0.143 | **MC2** | 0.062 | 0.98 |
| **MA4** | 0 | 0.015 | **MC3** | 0.024 | 0.67 |
| **MA5** | 0 | 0.277 | **MC4** | 0.163 | 2.54 |
| **MA6** | 0.036 | 0.842 | **MC5** | 0.054 | 1.40 |
| **MA7** | 0 | 0.040 | **MC6** | 0.004 | 0.83 |
| **MA8** | 0 | 0.059 | **MC7** | 0.179 | 2.29 |
| **MA9** | 0 | 0.035 | **MC8** | 0.040 | 1.20 |
| **MA10** | 0 | 0.396 | **MC9** | 0.081 | 1.72 |
| **MA11** | 0 | 0.030 | **MC10** | 0.025 | 2.84 |
| **FA1** | 0 | 0.000 | **FC1** | 0.092 | 1.53 |
| **FA2** | 0 | 0.086 | **FC2** | 0.051 | 0.96 |
| **FA3** | 0 | 0.061 | **FC3** | 0.050 | 0.91 |
| **FA4** | 0 | 0.055 | **FC4** | 0.052 | 1.79 |
| **FA5** | 0 | 0.107 | **FC5** | 0.052 | 1.50 |
| **FA6** | 0 | 0.000 | **FC6** | 0.007 | 0.72 |
| **FA7** | 0 | 0.745 | **FC7** | 0.023 | 1.09 |
| **Average** | 0.0021 | 0.2 | **Average** | 0.063 | 1.427 |
| **S.D.** | 0.0088 | 0.3 | **S.D.** | 0.049 | 0.638 |

| | | | | | |
|---|---|---|---|---|---|
| MA_ Male Alzheimer FA_ Female Alzheimer MC_ Male control FC_ Female control | | | | | |

### Industrial Applicability

There are provided a method for examining AD and a kit therefor.

## Claims

1. A method for examining Alzheimer's disease comprising:
(1) the measurement step of measuring an amount of lidocaine or a metabolite thereof in a cerebrospinal fluid derived from a lidocaine-administered test subject, and
(2) the determination step of determining whether the test subject has Alzheimer's disease or not on the basis of the measured amount.

2. The examination method according to claim 1, wherein the measurement step is performed by gas chromatography, liquid chromatography, immunoassay, or capillary electrophoresis.

3. The examination method according to claim 1, wherein the measurement step is performed by mass spectrometry.

4. The examination method according to claim 1, wherein the measurement step is performed by liquid chromatography and mass spectrometry.

5. An examination kit for Alzheimer's diseases for use in the examination method according to any one of claims 1 to 4, which comprises a reagent and/or an apparatus for quantifying lidocaine.
